# EUROPÄISCHE PATENTANMELDUNG

(11) **EP 2 289 513 A2**
(43) Veröffentlichungstag der Anmeldung: **02.03.2011**
(21) Anmeldenummer: 10184503.0
(22) Anmeldetag: 24.04.1997
(51) Int. Cl.: A61K 31/426, A61K 38/05, A61K 31/40, A61K 31/235, A61P 3/10

(54) **Verfahren zur Senkung des Blutglukosespiegels in Säugern**

(30) Priorität: 25.04.1996 DE 19616486
(62) Teilanmeldung aus: 00119496.8
(71) Anmelder: Prosidion Limited, Oxford, Oxfordshire OX4 6LT (GB)
(72) Erfinder: Demuth, Hans-Ulrich, 06114, Halle (DE); Rosche, Fred, 06184, Dieskau (DE); Schmidt, Jörn, 06114, Halle (DE); Pauly, Robert P., Edmonton Alberta AB T6G 2B7 (CA); McIntosh, Christopher H. S., Vancouver British Columbia V6T 1T7 (CA); Pederson, Ray A., Vancouver British Columbia V6S 1K9 (CA)
(74) Vertreter: Crump, Julian Richard John

(57) **Zusammenfassung**

Die Erfindung bezieht zur Verwendung eines Verfahrens, in dem durch das Verringern im Blut eines Säugers durch Leitung von effectors der Enzymaktivität der dipeptidyl Peptidase (DP IV) oder die Enzymaktivität ähnlich zu DP IV, das gastrische hemmende Polypeptid 1-42 (GIP1-42) des endogenen (oder zusätzlich exogen ausgeübt) insulinotropic Peptids und Glucagon-ähnliches Peptid amide-1 7-36 (GLP-17-36) (oder ähnlich GLP-17-37 oder Analogen davon) werden in einer verursachenden Sequenz in einem reduziert Umfang durch DP IV die Enzyme oder die zerlegt, die zu DP IV. ähnlich sind. Infolgedessen wird der Fall in die Konzentration der gesagten Peptid-Hormone oder die Analogen davon verringert oder verzögert.

Die erhöhte Stabilität, davon erzielt durch die Tätigkeit von DP IV effectors, des incretine oder der Analogen, die erhältlich endogen oder exogen und infolgedessen in den erhöhten Anzahlen für insulinotropic Anregung der incretine Rezeptoren der Langerhans Zellen in der Pankreas geliefert werden, ändert die Leistung des endogenen Insulins, dadurch siestimuliert siestimuliert den Metabolismus der Kohlenhydrate im behandelt Organismus. Der Blut-Zucker-Pegel fällt folglich unterhalb der Glucosekonzentration, die von der Hyperglykämie, im Serum des behandelt Organismus charakteristisch ist. Metabolische Abweichungen wie glucosuria, Hyperlipidemie, mögliche ernste metabolische Azidose, Diabetes mellitus, das Ergebnisse von den hocheren Konzentrationen der Glucose im Blut über einem längeren Zeitraum, verhindert oder vermindert werden.

Das Verfahren-gemäß der Erfindung ist eine neue Weise des Senkens der hohen Blut-Glucosekonzentrationen. Es ist einfach, kommerziell anwendbar und geeignet für Verwendung in der menschlichen Medizin für das Behandeln, insbesondere die Krankheiten, die durch über-durchschnittliche Blut-Glucose-Pegel verursacht werden.

## Beschreibung

Die Erfindung betrifft ein einfaches Verfahren zur Senkung der Blutzuckerkonzentration mit Hilfe von aktivitätsmindernden *Effektoren* (Substraten, Pseudosubstraten, Inhibitoren, Bindungsproteinen, Antikörpern u. a.) für Enzyme mit vergleichbarer oder identischer Aktivität zur enzymatischen Aktivität des Enzyms Dipeptidyl Peptidase IV.

Neben Proteasen, die in unspezifische Proteolyse einbezogen sind, was letztlich den Abbau von Proteinen zu Aminosäuren bewirkt, kennt man regulatorische Proteasen, die an der Funktionalisierung (Aktivierung, Deaktivierung, Modulierung) von endogenen Peptidwirkstoffen beteiligt sind [KIRSCHKE, H., LANGNER, J., RIEMANN, S., WIEDERANDERS, B., ANSORGE, S. and BOHLEY, P., Lysosomal cysteine proteases. Excerpta Medica (Ciba Foundation Symposium 75), 15 (1980); KRÄUSSLICH, H.-G. and WIMMER, E., Viral Proteinases. Ann. Rev. Biochem. 57, 701 (1987)]. Insbesondere im Zusammenhang mit der Immunforschung und der Neuropeptidforschung sind eine Reihe solcher sogenannten Konvertasen, Signalpeptidasen oder Enkephalinasen entdeckt worden [GOMEZ, S., GLUSCHANKOF, P., LEPAGE, A., MARRAKCHI, N. and COHEN, P., Proc. Natl. Acad. Sci. USA 85, 5468 (1988); ANSORGE, S. and SCHÖN, E., Histochem. 82, 41 (1987)].

Aufgrund der Häufigkeit des Vorkommens der Aminosäure Prolin in einer Vielzahl von Peptidhormonen und den damit verbundenen Struktureigenschaften dieser Peptide wird für prolinspezifische Peptidasen eine den Signalpeptidasen analoge Funktion diskutiert [YARON, A., The Role of Proline in the Proteolytic Regulation of Biologically Active Peptides. Biopolymers 26, 215 (1987); WALTER, R., SIMMONS, W.H. and YOSHIMOTO, T., Proline Specific Endo- and Exopeptidases. Mol. Cell. Biochem. 30, 111 (1980); VANHOOF, G., GOOSSENS, F., DE MEESTER, I., HENDRIKS, D. and SCHARPÉ, S., Proline motifs and their biological processing. FASEB Journal 9, 736 (1995)]. Dabei bestimmt Prolin in diesen Peptiden durch seine besondere Struktur sowohl Konformation als auch Stabilität dieser Peptide, indem sie vor Abbau durch unspezifische Proteasen schützt [KESSLER, H., Konformation und biologische Wirkung von zyklischen Peptiden. Angew. Chem. 94, 509 (1982)]. Enzyme, die dagegen hochspezifisch strukturverändernd auf Prolin-haltige Sequenzen einwirken (HIV-Protease, Cyclophylin u. a.) sind attraktive Ziele der aktuellen Wirkstoff-Forschung. Insbesondere für die nach dem Prolin spaltenden Peptidasen Prolyl Endopeptidase (PEP) und Dipeptidyl Peptidase IV (DP IV) konnten Beziehungen zwischen der Modulation der biologischen Aktivität von natürlichen Peptidsubstraten und deren selektiver Spaltung durch diese Enzyme wahrscheinlich gemacht werden. So nimmt man an, daß PEP eine Rolle beim Lernen bzw. im Gedächtnisprozeß spielt und DP IV in die Signalübertragung während der Immunantwort einbezogen ist [ISHIURA, S., TSUKAHARA, T., TABIRA, T., SHIMIZU, T., ARAHATA K. and SUGITA, H., *FEBS-Letters* 260, 131 (1990); HEGEN, M., NIEDOBI-TEK, G., KLEIN, C.E., STEIN, H. and FLEISCHER, B., *J*. *of Immunology* 144, 2908 (1990)].

Ähnlich wie die außerordentliche Prolinspezifität dieser Enzyme wird ihre hohe Selektivität für die Aminosäure Alanin innerhalb typischer Erkennungsregionen in Substraten dieser Enzyme diskutiert, wonach Alanin-haltige Peptide ähnliche Konfonnationen einnehmen können wie strukturanaloge Prolin-haltige Peptide. Kürzlich wurden derartige Eigenschaften Alaninhaltiger Peptidketten durch Punktmutation (Austausch von Prolin gegen Alanin) nachgewiesen [DODGE, R.W. and SCHERAGA, H.A., Folding and unfolding kinetics of the proline-toalanine mutants of bovine pancreatic ribonuclease A. *Biochemistry 35* (5) 1548 (1996)].

DP IV- bzw. DP IV-analoge Aktivität (z. B. besitzt die cytosolische DP II eine der DP IV nahezu identische Substratspezifität) kommt im Blutkreislauf vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen. Deshalb wird davon ausgegangen, daß dieses Enzym an der Regulation von Polypeptiden *in vivo* beteiligt ist [VANHOOF, G., GOOSSENS, F., DE MEESTER, I., HENDRIKS, D. and *SCHARPE,* S., Proline motifs and their biological processing, *FASEB Journal* 9, 736 (1995)].

Die Glukose-abhängigen insulinotropen Polypeptide: Gastric Inhibitory Polypeptide 1-42 (GIP₁₋₄₂) und Glucagon-Like Peptide Amide-1 7-36 (GLP-1₇₋₃₆), Hormone, die die Glukoseinduzierte Insulinsekretion des Pankreas stimulieren (auch *Incretine*), sind Substrate der DP IV, da sie von den N-terminalen Sequenzen dieser Peptide die Dipeptide Tyrosinyl-Alanin bzw. Histidyl-Alanin *in vitro* und *in situ* abspalten kann [MENTLEIN, R., GALLWITZ, B., and SCHMIDT, W.E., Dipeptidyl Peptidase IV hydrolyzes gastric inhibitory polypeptide, glucagon-like peptide-1 (7-36)amide, peptide histidine methionine and is responsible for their degradation in human serum. *Eur. J. Biochem.* 214, 829 (1993)].

Die Reduktion derartiger DP IV- bzw. DP IV-analoger Enzymaktivität zur Spaltung solcher Substrate *in vivo* kann dazu dienen, unerwünschte Enzymaktivität unter Laborbedingungen als auch in pathologischen Zuständen von Säuger-Organismen wirksam zu unterdrücken [DEMUTH, H.-U., Recent developments in the irreversible inhibition of serine and cysteine proteases. J. Enzyme Inhibition 3, 249-278 (1990); DEMUTH, H.-U. and HEINS, J., On the catalytic Mechanism of Dipeptidyl Peptidase IV. in Dipeptidyl Peptidase IV (CD 26) in Metabolism and the Immune Response (B. Fleischer, Ed.) R.G. Landes, Biomedical Publishers, Georgetown, 1-35 (1995)]. Z. B. basiert *Diabetes mellitus* Typ II (auch Altersdiabetes) auf einer verminderten Insulinsekretion bzw. Störungen in der Rezeptorfunktion, die u. a. in proteolytisch bedingten Konzentrationsanomalien der Incretine begründet sind [BROWN, J.C., DAHL, M., KWAWK, S., MCINTOSH, C.H.S., OTTE, S.C. and PEDERSON, R.A. Peptides 2, 241 (1981); SCHMIDT, W.E., SIEGEL, E.G., GALLWITZ, B. KUMMEL, H., EBERT, R. and CREUTZFELDT, W., Characterization of the inulinotropic activity of fragments,derived from gastric inhibitory polypeptide. Diabetologia 29, 591A (1986); ADELHORST, K., HE-DEGAARD, B.B., KNUDSEN, L.B. and KIRK, O., Structure-activity studies of glucagon-like peptide. J. Biol. Chem. 296, 6275 (1994)].

Hyperglykämie und damit verbundene Ursachen bzw. Folgeerscheinungen (auch *Diabetes mellitus*) werden nach gegenwärtigem Stand der Technik durch die Verabreichung von Insulin (z.B. von aus Rinderpankreas isoliertem oder auch gentechnisch gewonnenem Material) an erkrankte Organismen in verschiedenen Darreichungsformen behandelt. Alle bisher bekannten, als auch die moderneren Verfahren zeichnen sich durch hohen Materialaufwand, hohe Kosten und oft durch entscheidende Beeinträchtigungen der Lebensqualität der Patienten aus. Die klassische Methode (tägliche *i.v..* Insulin-Injektion, üblich seit den dreißiger Jahren) behandelt die akuten Krankheitssymptome, führt aber nach längerer Anwendung u. a. zu schweren Gefäßveränderungen (Arteriosklerose) und Nervenschädigungen [LACY, P., Status of Islet Cell Transplantation. Diabetes Care 16 (3) 76 (1993)].

Neuerdings wird die Installation subkutaner Depot-Implantate (die Insulinabgabe erfolgt dosiert, und die täglichen Injektionen entfallen) sowie die Implantation (Transplantation) intakter Langerhansscher Zellen in die funktionsgestörte Pankreasdrüse oder andere Organe und Gewebe vorgeschlagen. Derartige Transplantationen sind technisch aufwendig. Weiterhin stellen sie einen risikobehafteten chirurgischen Eingriff in den Empfängerorganismus dar und verlangen auch bei Zellverpflanzungen nach Methoden zur Suppression bzw. der Umgehung des Immunsystems [LACY, P., Treating Diabetes with Transplanted Cells. Sci. Americ. 273 (1) 40-46 (1995)].

Die möglichst orale Applikation hochaffiner, niedermolekularer Enzyminhibitoren dagegen ist eine kostengünstigere Alternative z. B. zu invasiven chirurgischen Techniken bei der Behandlung pathologischer Erscheinungen. Derartige Enzyminhibitoren finden inzwischen therapeutischen Einsatz als Immunsuppressiva, Antithrombotika und als *AIDS*-Virostatika. Durch chemisches Design von Stabilitäts-, Transport- und Clearence-Eigenschaften kann deren Wirkungsweise modifiziert und auf individuelle Eigenschaften abgestimmt werden [SANDLER, M. and SMITH, H.J., Hrsg., Design of Enzyme Inhibitors as Drugs. Oxford University Press, Oxford (1989); MUNROE, J.E., SHEPHERD, T.A., JUNGHEIM, L.N., HORNBACK, W.J., HATCH, S.D., MUESING, M.A., WISKERCHEN, M.A., SU, K.S., CAMPANALE, K.M., BAXTER, A.J., and COLACINO, J.M., Potent, orally bioavailable HIV-1 protease inhibitors containing noncoded D-amino acids. Bioorg. Medicinal Chem. Letters 5 (23) 2897 (1995)].

Das Ziel der Erfindung ist ein einfaches und neuartiges Verfahren zur Senkung des Blutglukosespiegels, das erfindungsgemäß dadurch erreicht werden kann, daß mittels Verabreichung von Effektoren an einen Säugerorganismus, in kausaler Folge die endogenen (oder zusätzlich exogen verabreichten) insulinotropen Peptide GIP₁₋₄₂ und GLP-1₇₋₃₆ (o.a. GLP-1₇₋₃₇ oder deren Analoga) durch DP IV- oder DP IV-ähnliche Enzyme vermindert abgebaut werden und damit die Konzentrationsabnahme dieser Pepdidhormone bzw. ihrer Analoga verringert bzw. verzögert wird.

Der Erfindung liegt der überraschende Befund zugrunde, daß eine Reduktion der im Blutkreislauf agierenden DP IV- oder DP IV-ähnlichen enzymatischen Aktivität kausal zur Beeinflussung des Blutzuckerspiegels führt. Es wurde gefunden, daß
1. die Verminderung von DP IV- bzw. DP IV-analoger Aktivität zu relativer Stabilitätserhöhung der Glukose-stimulierten, oder extern zugeführten Incretine (oder deren Analoga) zur Folge hat, d.h. durch Applikation von Effektoren der DP IV bzw. DP IV-anloger Proteine der Incretin-Abbau im Blut kontrolliert werden kann.
2. erhöhte biologische Abbaustabilität der Incretine (oder ihrer Analoga) eine Wirkungsveränderung endogenen Insulins zur Folge hat.
3. die durch Reduktion der DP IV- bzw. DP IV-analogen enzymatischen Aktivität im Blut erzielte Stabilitätserhöhung der Incretine in nachfolgender Veränderung der Glukoseinduzierten Insulinwirkung resultiert und damit zu einer mittels DP IV-Effektoren kontrollierbaren Modulierung des Blut-Glukosespiegels führt.

Die Erfindung betrifft somit die Verwendung von Effektoren der Dipeptidyl Peptidase VI (DP IV)- bzw. DP IV-analoger Enzymaktivität. Zur Senkung des Blutzuckerspiegels unter die für Hyperglykaemie charakteristische Glukosekonzentration im Serum eines Säuger-Organismus. Insbesondere betrifft die Erfindung die Verwendung von Effektoren der DP IV- bzw. der DP IV-analogen Enzymaktivität an Säugern der Verhinderung oder Milderung pathologischer Stoffwechsel-Anomalien von Säuger-Organismen ausgewählt aus Glukosurie, Hyperlipidaemie, metabolischer Azidosen und Diabetes Mellitus. In einer weiteren bevorzugten Ausfuehrungsform betrifft die Erfindung ein Verfahren zur Senkung des Blutzuckerspiegels unter die für Hyperglykaemie charakteristische Glukosekonzentration im Serum eines SäugerOrganismus, das dadurch gekennzeichnet ist, daß man einem Säuger-Organismus eine therapeutisch wirksame Menge eines Effektos der DP IV- bzw. der DP IV-analogen Enzymaktivität verabreicht.

In einer zweiten bevorzugten Ausführungsform betrifft die Erfindung Effektoren der DP IV- bzw. der DP IV-Analogen Enzymaktivität zur Anwendung in einem Verfahren zur Senkung des Blutzucker-Spiegels unter die für Hyperglykaemie charakteristische Glukose-Konzentration im Serum eines Säuger-Organismus.

Die erfindungsgemäß applizierten Effektoren der DP IV- bzw. DP IV-analoger Enzyme können in pharmazeutisch anwendbaren Formulierungskomplexen als Inhibitoren, Substrate, Pseudosubstrate, Inhibitoren der DP IV-Expression, Bindungsproteine oder Antikörper dieser Enzymproteine oder Kombinationen aus diesen verschiedenen Stoffen, die DP IV- bzw. DP IV-analoge Proteinkonzentration im Säugerorganismus reduzieren, zum Einsatz kommen. Erfindungsgemäße Effektoren sind z.B. DP IV-Inhibitoren wie die Dipeptidderivate bzw. Dipeptidmimetika Alanyl-Pyrolidid, Isoleucyl-Thiazolidid sowie das Pseudosubstrat N-Valyl-Prolyl, O-Benzoyl Hydroxylamin. Derartige Verbindungen sind aus der Literatur bekannt [DEMUTH, H.-U., Recent developments in the irreversible inhibition of serine and cysteine proteases. J. Enzyme Inhibition 3, 249 (1990)] oder in Analogie zu den in der Literatur beschriebenen Methoden herstellbar.

Das erfindungsgemäße Verfahren stellt eine neuartige Herangehensweise zur Senkung erhöhter Blutglukosekonzentration im Serum von Säugern dar. Es ist einfach, kommerziell nutzbar und zur Anwendung bei der Therapie, insbesondere von Erkrankungen, die auf überdurchschnittlichen Blutglukosewerten basieren, in der Humanmedizin geeignet.

Die Effektoren werden in Form von pharmazeutischen Präparaten enthaltend den Wirkstoff in Kombination mit üblichen aus dem Stand der Technik bekannten Trägermaterialien verabreicht. Beispielsweise werden sie parenteral (z.B. *i.v.,* in physiologischer Kochsalzlösung) oder enteral (z.B. oral, formuliert mit üblichen Trägermaterialien wie z. B. Glukose) appliziert.

In Abhängigkeit von ihrer endogenen Stabilität und ihrer Bioverfügbarkeit müssen einfache oder auch mehrfache Gaben der Effektoren erfolgen, um die erwünschte Normalisierung der Blutglukosewerte zu erreichen. Z. B. kann im Falle von Aminoacyl-Thiazolididen ein solcher Dosisbereich zwischen 1.0 mg und 10.0 mg Effektorsubstanz pro Kilogramm liegen.

### Ausrührungsbeispiele

### Beispiel 1: Inhibierung der DP IV-katalysierten Hydrolyse der Incretine GIP₁₋₄₂ und GLP-1₇₋₃₆ in situ

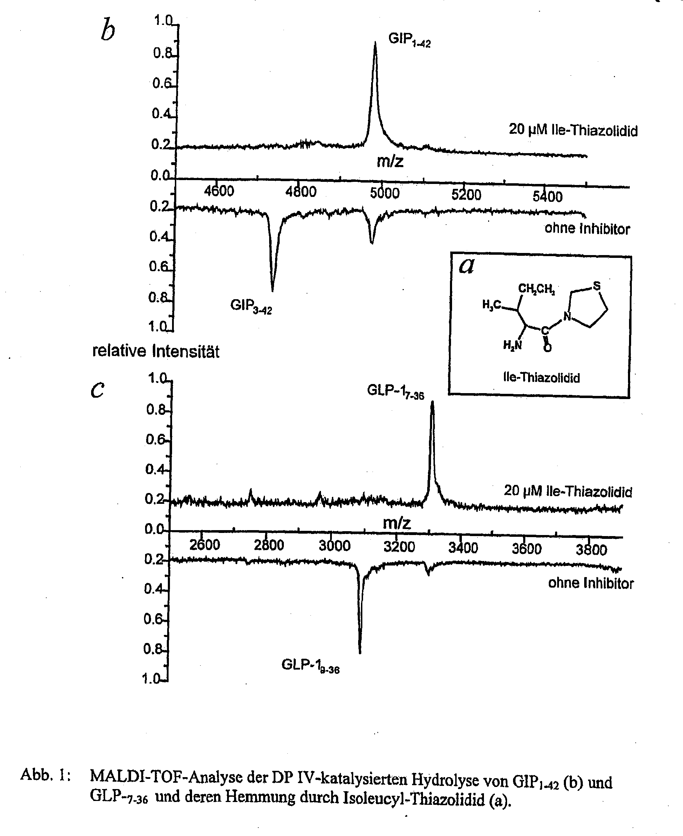

Sowohl *in vitro* mit gereinigtem Enyzm als auch *in situ,* z.B. in gepooltem humanem Serum, kann man die Hydrolyse der Incretine, verursacht durch DP IV- bzw. DP IV-analoge Aktivität, nachweisen bzw. mit Hilfe von Inhibitoren unterdrücken (Abb. 1).

Erfindungsgemäß erreicht man *in situ* bei Inkubation von 30 µM GIP₁₋₄₂ bzw. 30 µM GLP-1₇₋₃₆ und 20 µM Isoleucyl-Thiazolidid (1a), einem reversiblen DP IV-Inhibitor in 20 %-igem Serum bei pH 7.6 und 30 °C die komplette Unterdrückung der Enzym-katalysierten Hydrolyse beider Peptid-hormone innerhalb von 24 Stunden (1b und 1c, jeweils obere Spektren. Synthetisches GIP₁₋₄₂ (5 µM) und synthetisches GLP-1₇₋₃₆ (15 µM) wurden mit humanem Serum (20 %) in 0.1 mM TRICINE Puffer bei pH 7.6 und 30°C für 24 Stunden inkubiert. Proben der Inkubationsansätze (für GIP₁₋₄₂ 2.5 pmol und im Falle von GLP-1₇₋₁₆ 7.5 pmol) wurden nach verschiedenen Zeiten entnommen. Die Proben wurden mit 2',6'-Dihydroxyacetophenon als Matrix co-kristallisiert und mittels MALDI-TOF-Massen-spektrometrie analysiert. Die Spektren (Abb. 1) stellen Akkumulationen von 250 einzelnen Laserschüssen pro Probe dar.

(1b) Die Signale im Bereich von *mlz* 4980.1 ± 5.3 entsprechen GIP1₋₄₂ (*M* 4975.6) und *m*/*z* 4745.2 ±5.5 dem DP IV-Hydrolyseprodukt GIP₃₋₄₂ (M 4740.4).

(1c) Die Signale *m*/*z* 3325.0 ± 1.2 entsprechen GLP-1₇₋₃₆ (M 3297.7) und *m*/*z* 3116.7 ± 1.3 dem DP IV-Hydrolyseprodukt GLP-1₉₋₃₆ (M 3089.6).

In den Versuchsansätzen ohne Inhibitor wurden die Incretine in dieser Zeit fast vollständig abgebaut (Abb. 1b und 1c, jeweils untere Spektren).

### Beispiel 2: Inhibierung des Abbaus von GLP-1₇₋₃₆ durch den DP IV-Inhibitor Isoleucyl-Thiazolidid in vivo.

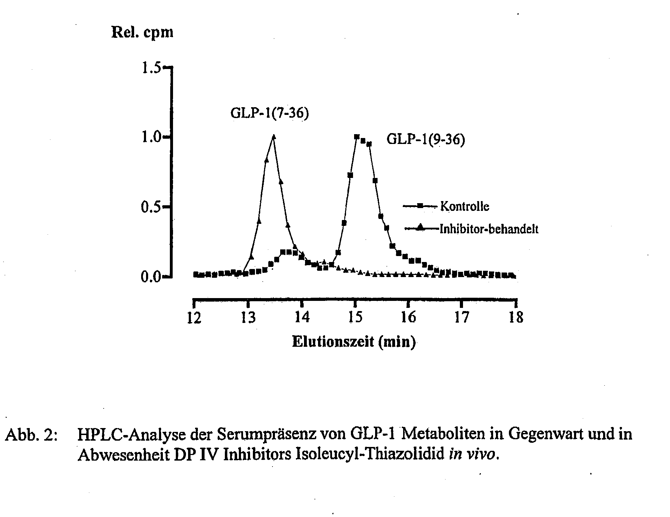

Verfolgt man den Metabolismus der nativen Incretine (hier GLP-1₇₋₃₆) im Serum der Ratte in Abhängigkeit in Gegenwart des DP IV-Inhibitors Isoleticyl-Thiazolidid (*i.v.* Injektion einer 1.5 µM Inhibitorlösung in 0.9 %-iger Kochsalzlösung) gegenüber einer Kontrolle, so ist bei einer Konzentration des Inhibitors Isoleucyl-Thiazolidid von ca. 0.1 mg/kg Laborratte bei den Inhibitor-behandelten Versuchstieren (n = 5) im Verlaufe des Versuchszeitraums kein Abbau des insulino-tropen Peptidhormons CLP-1₇₋₃₆ zu beobachten (Abb. 2).

Zur Detektion der Metaboliten in Anwesenheit und Abwesenheit des DP IV-Inhibitors (20 Minuten nach vorheriger *i.v*.-Inhibitor- bzw. Kochsalzgabe) erhielten die Versuchs- und Kontrolltiere *i.v.* 50 - 100 pM ¹²⁵I-GLP-1₇₋₃₆ (spezifische Aktivität ca. 1 µMCi/pM). Blutproben wurden nach 2 - 5 min entnommen und das Plasma mittels 20 % Acetonitril extrahiert. Nachfolgend wurde der Peptidextrakt mittels RP-HPLC separiert und die Radioaktivität der Fraktionen an einem γ-Counter analysiert. Die gefundene Aktivität ist in cpm (*counts per minute* relativ zum Maximum angegeben).

### Beispiel 3: Modulation der Insulinwirkung und Senkung des Blutglukosespiegels nach i.v. Applikation des DP IV-Inhibitors Isoleuzyl-Thiazolidid in vivo.

An der durch intraduodenale (*i.d*.) Injektion Glukose-stimulierten Ratte, kann durch *i.v.* Gabe verschiedener DP IV-Effektoren, z. B. von 0.1 mg Isoleucyl-Thiazolidid pro kg Ratte eine auf die Inhibitorwirkung zurückgehende, zeitlich verzögert einsetzende Senkung des Glukosespiegels beobachtet werden. Dieser Effekt ist dosisabhängig und nach Absetzen der Infusion von 0.05 mg/min des DP IV-Inhibitors Isoleucyl-Thiazolidid pro kg Ratte reversibel. Die *i.v.* Applikation der gleichen Glukosemenge von Inhibitor-behandelten und Kontroll-Tieren zeigt im Gegensatz zur den *i.d.* Glukose-stimulierten Versuchstieren keine vergleichbare Wirkung,

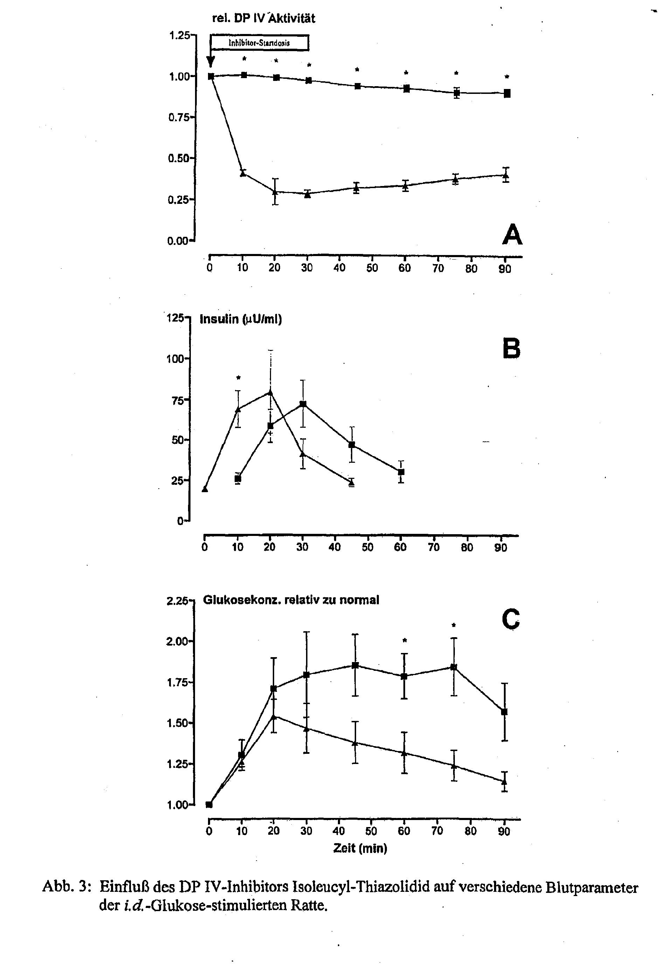

Abbildung 3 verdeutlicht diese Zusammenhänge an den Inhibitor-abhängigen Veränderungen der Plasmaparameter: A - DP IV-Aktivität, B - Plasma-Insulinspiegel, C - Blutglukosespiegel.

Die Versuchstiere (n = 5, männliche Wistar-Ratten, 200-225 g) erhielten als Initialdosis 1.5 µm Isoleucyl-Thiazolidid in 0.9 %-iger Kochsalzlösung (▲) oder gleiche Volumina 0.9%-ige Kochsalzlösung ohne Inhibitor (■) (Kontrollgruppe n = 5). Die Versuchsgruppe erhielt weiterhin eine Infusion des Inhibitors von 0.75 µM/min über 30 min Versuchszeit (*). Der Kontrollgruppe wurde im gleichen Zeitraum eine Inhibitor-freie 0.9%-ige Kochsalzlösung infundiert. Zum Zeitpunkt t=0 erhielten die Tiere *i.d.* eine Glukosedosis von 1g/kg 40%iger Dextroselösung (w/v).

Allen Versuchstieren wurden Blutproben in zehn Minutenabständen entnommen.

Glukose Messungen erfolgten am Vollblut (Lifescan One Touch II analyzer) während die DP IV-Aktivität und die Insulinkonzentrationen im Plasma bestimmt wurden.

Der hier angewandte Insulintest ist empfindlich zwischen 10 und 160 mU/ml [PEDERSON, R.A., BUCHAN, A.M.J., ZAHEDI-ASH, S., CHEN, C.B. & BROWN, J.C. Reg. Peptides. 3, 53-63 (1982)]. Die DP IV-Aktivität wurde spektralphotometrisch bestimmt [DEMUTH, H.-U. and HEINS, J., On the catalytic Mechanism of Dipeptidyl Peptidase IV. in Dipeptidyl Peptidase IV (CD 26) in Metabolism and the Immune Response (B. Fleischer, Ed.) R.G. Landes, Biomedical Publishers, Georgetown, 1-35 (1995)]. Alle Meßwerte sind als Mittelwerte mit Standardabweichung angegeben.

## Patentansprüche

1. Verwendung von Effektoren der Dipeptidyl Peptidase (DP IV)- bzw. DP IV-analoger Enzymaktivität zur Senkung des Blutzuckerspiegels unter die für Hyperglykämie charakteristische Glukosekonzentration im Serum eines Säuger-Organismus,

2. Verwendung gemäß Anspruch 1, **dadurch gekennzeichnet, daß** die Verabreichung von Effektoren der DP IV- bzw. der DP IV-analogen Enzymaktivität an Säuger der Verhinderung oder Milderung pathologischer Stoffwechsel-Anomalien von Säuger-Organismen ausgewählt aus Glukosurie, Hyperlipidämie, metabolischer Azidosen und *Diabetes mellitus* dient.

3. Verwendung gemäß Anspruch 1, **dadurch gekennzeichnet, daß** als Effektoren der Dipeptidyl Peptidase (DP IV)- bzw. DP IV-analoger Enzymaktivität Inhibitoren, Substrate, Pseudosubstrate, Inhibitoren der DP IV-Expression, Bindungsproteine oder Antikörper dieser Enzymproteine oder Kombinationen der genannten Effektoren verwendet werden.

4. Effektoren der DP IV- bzw. der DP IV-analogen Enzymaktivität zur Anwendung in einem Verfahren zur Senkung des Blutzucker-Spiegels unter die für Hyperglykämie charakteristische Glukose-Konzentration im Serum eines Säuger-Organismus.
